# EUROPÄISCHE PATENTSCHRIFT

(11) **EP 4 583 781 B1**
(45) Veröffentlichungstag und Bekanntmachung des Hinweises auf die Patenterteilung: **20.05.2026**
(21) Anmeldenummer: 23758620.1
(22) Anmeldetag: 18.08.2023
(51) Int. Cl.: A61B 5/243, A61B 5/055

(54) **VORRICHTUNG ZUM ERFASSEN VON MAGNETISCHEN SIGNALEN, DIE VON EINEM SCHLAGENDEN HERZ ERZEUGT WERDEN**
DEVICE FOR DETECTING MAGNETIC SIGNALS GENERATED BY A BEATING HEART
DISPOSITIF DE DÉTECTION DE SIGNAUX MAGNÉTIQUES GÉNÉRÉS PAR UN COEUR BATTANT

(30) Priorität: 09.09.2022 DE 102022209424
(43) Veröffentlichungstag der Anmeldung: 16.07.2025
(73) Patentinhaber: Robert Bosch GmbH, 70442 Stuttgart (DE)
(72) Erfinder: DOLDE, Florian, 70599 Stuttgart (DE); SCHAAL, Frederik, 72770 Reutlingen (DE); KRIST, Florian, 70190 Stuttgart (DE); CIPOLLETTI, Riccardo, 73312 Geislingen An Der Steige (DE); WEHRSE, Eckhard, 70563 Stuttgart (DE)
(86) Internationale Anmeldenummer: PCT/EP2023/072816
(87) Internationale Veröffentlichungsnummer: WO 2024/052085

(56) Entgegenhaltungen:
- WO-A1-2020/120924
- US-A1- 2022 015 667
- US-A1- 2022 228 998
- MASUYAMA YUTA ET AL: "Gradiometer Using Separated Diamond Quantum Magnetometers", SENSORS, vol. 21, no. 3, 2 February 2021 (2021-02-02), pages 977, XP055954870, DOI: 10.3390/s21030977
- BLAKLEY S. M. ET AL: "Room-temperature magnetic gradiometry with fiber-coupled nitrogen-vacancy centers in diamond", OPTICS LETTERS, vol. 40, no. 16, 5 August 2015 (2015-08-05), US, pages 3727, XP055954812, ISSN: 0146-9592, DOI: 10.1364/OL.40.003727

## Beschreibung

Die vorliegende Erfindung betrifft eine Vorrichtung zum Erfassen von magnetischen Signalen, die von einem schlagenden Herz erzeugt werden

### Hintergrund der Erfindung

Um sehr kleine Magnetfeldstärken zu messen, eignen sich als Sensoren insbesondere optisch gepumpte oder auf NV-Zentren in Diamant basierende Quantensensoren. In der DE 10 2022 204 526.2 wird ein Magnetometer beschrieben, das optisch gepumpte und optisch detektierte magnetische Resonanzen (optically detected magnetic resonance, ODMR) nutzt. Dabei wird ausgenutzt, dass unter Einfluss eines äußeren Magnetfelds die Energieniveaus bestimmter Spinzustände ungepaarter Elektronen aufspalten, der sogenannte Zeeman-Effekt. Durch die Aufspaltung der Energieniveaus ergeben sich veränderte Übergänge bei der Relaxation aus angeregten Zuständen, die dann beispielsweise durch optische Anregung und frequenzabhängige Detektion der resultierenden Fluoreszenzstrahlung oder durch Beobachtung optischer Eigenschaften wie der Absorption von Licht gemessen werden können. Aus den gemessenen optischen Parametern kann dann wiederum auf die Magnetfeldstärke geschlossen werden.

Die US-Patentanmeldung US2022/015667 A1 beschreibt Systeme und Methoden zur Überwachung der Atmung und Herzfunktion eines Patienten durch einen Sensor, der für die Integration in ein Bett, einen Stuhl oder eine Matratze konzipiert oder direkt am Patienten anzubringen ist. Bei dem Sensor kann es sich u.a. um Magnetometer zur Erfassung von Änderungen in magnetischen Feldern handeln.

Die Veröffentlichung "Gradiometer Using Separated Diamond Quantum Magnetometers" von Masuyama Yuta et al. Bd. 21, Nr. 3, 2.februar 2021 (2021-02-02), Seite 977 beschreibt ein Gradiometer, das zwei Diamant-Quantenmagnetometer mit negativ geladenen Stickstoff-Fehlstellen (NV-Zentren) umfasst. Das Gradiometer ist in der Lage, Umgebungsrauschen, insbesondere bei niedrigen Frequenzen, zu unterdrücken, wodurch hochsensitive Magnetfeldmessungen ohne die Notwendigkeit einer magnetischen Abschirmung möglich werden.

### Offenbarung der Erfindung

Erfindungsgemäß wird eine Vorrichtung zum Erfassen von magnetischen Signalen, die von einem schlagenden Herz erzeugt werden, mit den Merkmalen des Anspruchs 1 vorgeschlagen. Vorteilhafte Ausgestaltungen sind Gegenstand der Unteransprüche sowie der nachfolgenden Beschreibung.

Ein Magnetokardiogramm (abgekürzt MKG) ist die Aufnahme und Darstellung des Magnetfeldes des Herzes, das durch die elektrophysiologische Aktivität der Herzmuskelzellen entsteht. Im Rahmen der Erfindung wird eine kontaktlose, passive Möglichkeit der Langzeitüberwachung des menschlichen Herzes mit hoher Auflösung vorgestellt. Dies wird durch Stickstoff-Fehlstellen-Magnetometer (sog. NV-Magnetometer) in einer geometrischen Anordnung realisiert.

Im Einzelnen wird nun eine Vorrichtung zum Erfassen von magnetischen Signalen, die von einem schlagenden Herz erzeugt werden, vorgestellt, welche einen Unterlagekörper mit einer Auflagefläche und eine Anordnung aus wenigstens zwei NV-Magnetometereinheiten aufweist, wobei die Anordnung in dem Unterlagekörper eingebettet ist, wobei der Unterlagekörper dazu eingerichtet ist, einen Benutzer sitzend oder liegend auf der Auflagefläche aufzunehmen. Eine solche Vorrichtung kann auch als Magnetokardiograf bezeichnet werden.

Ein besonderer Vorteil der NV-Sensorik ist deren Größe, speziell des Sensormediums. Für die Anwendung sollte das aktive Messvolumen klein gegenüber dem zu messenden Objekt (Herz) sein, da ansonsten durch die Flächenabdeckung über große Teile des Signals integriert wird und somit das Signal ggf. verschwindet, da das Integral null ist. Je kleiner das aktive Messvolumen im Vergleich zum Herz, desto besser ist die Signaldetektion. NV-Sensorik hat ein sehr kleines aktives Sensorvolumen. Diese Kleinbaubarkeit ermöglicht zudem die Verwendung der Sensoren in einer geometrischen Anordnung. Insbesondere sind sehr hochauflösende Anordnungen durch das sehr kleine aktive Sensorvolumen möglich.

Dies ermöglicht auch die einfache Integration in Textilien oder andere Alltagsgegenstände, wobei zahlreiche Optionen angedacht sind. In einer Ausgestaltung ist der Unterlagekörper ein Polster, eine Matratze, eine Liege, eine Matte, ein Bett, ein Sitz (wie z.B. Autositz) oder ein Stuhl; eine Integration ist auch möglich in z.B. Topper, Unterleger, Überzug, Lattenrost, Bettgestell, Bettdecke, Kissen, Seitenschläferkissen usw.

Diamant-NV-Magnetometer beruhen auf dem Auslesen der Magnetresonanzen von speziellen Defektzentren in Diamant, insbesondere von Stickstoff-Fehlstellen (NV, nitrogen vacancy), die als Verunreinigungen des Kohlenstoffgitters von Diamant auftreten und auch gezielt eingebracht werden können. Wird das NV-Zentrum im Grundzustand optisch angeregt, indem z.B. ein Pumplaserstrahl mit geeigneter Wellenlänge (in diesem Fall im grünen Wellenlängenbereich, z.B. bei 532nm für eine off-resonance-Anregung) eingestrahlt wird, werden die Elektronen vom Triplett-Grundzustand in den angeregten Triplett-Zustand gehoben und relaxieren unter Emission von Fluoreszenzlicht im roten Wellenlängenbereich bei 650 - 800 nm (637nm = zero phonon line). Da die Wahrscheinlichkeit für nicht spinerhaltende Übergänge aus dem Spinzustand mit der Spinquantenzahl mₛ=±1 größer ist, sorgt ein fortlaufendes Anregungspumpen dafür, dass die NV-Zentren größtenteils im Spinzustand mₛ=0 hyperpolarisiert werden.

Zwischen den mₛ = 0 und mₛ=±1 Spinzuständen im Grundzustand besteht eine Energiedifferenz, die in diesem Fall bei etwa 2,87 GHz liegt. Strahlt man also neben der optischen Anregung noch Mikrowellenstrahlung in den Diamanten ein, kommt es bei dieser Resonanzfrequenz von 2,87 GHz zu einem Einbruch der roten Fluoreszenz, da die spinpolarisierten Elektronen durch das Mikrowellenfeld vom mₛ = 0 in den mₛ=±1 -Grundzustand gehoben werden und von dort durch das Pumplicht in den mₛ=±1 angeregten Zustand angeregt werden. Von dort treten jedoch vor allem nichtstrahlende Übergänge und schwach infrarote Fluoreszenzübergänge über den Singulett-Zustand auf, während die Fluoreszenz im roten Bereich wegfällt.

Wenn nun ein externes Magnetfeld vorhanden ist, kommt es durch den sogenannten Zeeman-Effekt zur Aufspaltung der ansonsten gleichenergetischen mₛ=±1 Triplett-Niveaus in energetisch äquidistante Zeeman-Niveaus. Bei Auftragung der Fluoreszenz gegen ein Frequenzspektrum der Mikrowellenanregung zeigen sich dann zwei Dips im Fluoreszenzspektrum, deren Frequenzabstand proportional zur magnetischen Feldstärke des externen Magnetfelds ist. Die Magnetfeldsensitivität wird dabei vor allem durch die minimal auflösbare Frequenzverschiebung definiert und kann bis 1 pT/√Hz oder weniger erreichen. Da das NV-Zentrum im einkristallinen Diamanten vier Möglichkeiten besitzt, sich im Kristallgitter anzuordnen, kommt es bei Anwesenheit eines gerichteten Magnetfelds dazu, dass die im Kristall vorhandenen NV-Zentren je nach Lage im Kristall unterschiedlich stark auf das äußere Magnetfeld reagieren. Dadurch können im Idealfall vier Paare von Fluoreszenz-Minima im Spektrum auftauchen, aus deren Form und Lage zueinander sowohl die Magnetfeldstärke als Betrag als auch die Richtung des externen Magnetfelds eindeutig bestimmbar sind.

Um vektorielle Magnetfeldmessungen zu ermöglichen, weist die Vorrichtung in einer Ausgestaltung eine Einrichtung zum Erzeugen eines im Wesentlichen homogenen Bias-Magnetfelds im Bereich der Magnetometereinheiten bzw. deren Sensormedien auf. Die Einrichtung kann ebenfalls in dem Unterlagekörper integriert sein. Es kann sich dabei um eine Helmholtz-Spulenanordnung handeln, wobei mindestens das Sensormedium der wenigstens zwei NV-Magnetometereinheiten innerhalb der Helmholtz-Spulenanordnung angeordnet ist. Es kann sich ebenso um andere Einrichtungen handeln wie z.B. eine einfache Spule, eine langgezogene Spule, Permanentmagnetlösungen wie z.B. in einem Hallbacharray usw.

Herzsignale haben in einigen cm Abstand eine magnetische Signatur mit einer Amplitude von (nur noch) 1 bis 2-stelligen Picotesla (pT), wohingegen z.B. das Erdmagnetfeld in Mitteleuropa ca. 50 µT (Mikrotesla) beträgt, also um einen Faktor 10⁶ stärker ist. Selbst so kleine Feldstärken sind jedoch mit der vorgeschlagenen Technologie langzeitig hochgenau auflösbar. Beispielsweise kann dazu eine magnetische Abschirmung oder eine Gradiometerverschaltung verwendet werden.

Durch die hochauflösende Detektion des genauen Herzsignals kann eine Vielzahl an Krankheiten detektiert werden, wie beispielsweise permanentes Vorhofflimmern und anfallsweises ("Paroxysmales") Vorhofflimmern. Somit kann einem Herzinfarkt und in der Folge einem Schlaganfall (insbesondere nach unerkanntem Herzinfarkt) vorgebeugt werden. Ferner eignet sich die Erfindung zur Früherkennung eines S-T-Hebungsinfarkts, andersartigen Hebungsinfarkts, einer Lungenembolie, einer AV-Knoten-Rentry-Tachykardie, von ventrikuläre Extrasystolen, aber auch sehr seltene pathogene Erkrankungen wie z.B. einer arrhythmogenen rechtsventrikulären Tachykardie, die sonst nur durch eine Gensequenzierung erkannt werden können.

Erst eine genaue Auflösung des Herzsignals ermöglicht diese Detektion der Krankheitsbilder. Im Fall stark verrauschter oder schlecht aufgelöster Signale sind nämlich die Verschiebungen der verschiedenen PQRST-Komplexe des Herzes gegeneinander bzw. über die Zeit, Schwankungen in deren Amplitude, Verformungen oder kleine Störungen nicht detektierbar. Diese Auflösung dieser Kriterien sind allerdings wichtige Faktoren, da die oben genannten Problematiken zu Vertauschungen von Komplexen (z.B. Interpretation von erhöhter und verschobener T-Welle als R-Welle, was allerdings bei einem "gesunden" Herz häufig vorkommt) und zu Fehlalarmen führen kann.

Wenn eine Gradiometerverschaltung der wenigstens zwei NV-Magnetometereinheiten verwendet wird, hat immer eine Magnetometereinheit einen größeren Abstand zum Herz (als relativ schwache Magnetfeldquelle) als eine andere Magnetometereinheit. Durch die Gradiometerverschaltung, d.h. im Wesentlichen (vektorielle) Subtraktion des Gemessenen, entspricht der Magnetfeldgradient näherungsweise dem Feld, das von der schwachen Quelle ausgeht, während wesentlich stärkere Hintergrundfelder (die in beiden Magnetometereinheiten im Wesentlichen gleich sind) eliminiert werden. Damit entfällt die Notwendigkeit einer magnetischen Abschirmung, so dass die Magnetfeldmessung in Alltagsumgebungen möglich wird. Die Erfindung eignet sich entsprechend insbesondere zur nichtabgeschirmten Messung schwacher Magnetfelder. Technische Details zu Gradiometerlösungen, die auch im Rahmen der vorliegenden Erfindung angewendet werden können, sind in der DE 102022201690.4 offenbart, und sollen hier einbezogen sein.

Die NV-Sensorik wird zweckmäßigerweise so integriert, dass sie nicht spürbar bzw. nicht störend ist. In einer Ausgestaltung weist der Unterlagekörper elastisches Material zwischen der Anordnung und der Auflagefläche auf. Durch die Wahl von speziellen Materialien hinsichtlich Wärmeleitfähigkeit, Elastizität, Härte usw. kann auch eine gewisse Temperaturkontrolle bzw. Druckkontrolle erzielt werden, so dass die NV-Einheiten nicht beschädigt und Personen nicht verletzt werden.

In einer Ausgestaltung ist wenigstens eine Struktur aus einem Material mit hoher magnetischer Permeabilität »1, z.B. größer 10, 100 oder 1000, insbesondere ferromagnetisches Material, z.B. enthaltend Eisen, Kobalt, Nickel, auf einer der Auflagefläche abgewandten Seite der Anordnung und/oder in einem (zusätzlichen) Auflagekörper vorgesehen. Dies kann zur Feldführung bzw. Abschirmung (z.B. von anderen Magnetfeldern oder Mikrowellen) dienen. Insbesondere kann die Struktur eine Schicht, z.B. Platte oder Folie, eine Gitterstruktur, z.B. ein Netz, usw. aufweisen. Ist die Struktur auf einer der Auflagefläche abgewandten Seite der Anordnung vorgesehene, kann sie unter dem Unterlagekörper angeordnet oder im Unterlagekörper eingebettet sein. Der Auflagekörper kann ein sog. Topper oder eine Zudecke sein.

In einer Ausgestaltung ist die Vorrichtung dazu eingerichtet, mittels jeder der wenigstens zwei NV-Magnetometereinheiten eine magnetische Feldstärke und Feldrichtung zu erfassen. Ein weiterer Vorteil der NV-Sensorik ist die Richtungs- bzw. Vektorinformation. Im Gegensatz zu weiteren Technologien ist diese bei NV-Sensorik intrinsisch gegeben. Es müssen also weder durch Modulationstechniken Störungen eingeführt bzw. ungünstigere Projektionen genutzt werden, noch mehrere separate Sensoren verwendet werden. Man hat somit die Vektor- und Gradiometrieinformation am exakt selben Ort (Diamantgröße, also einstellige mm^3 und darunter) und nicht einige cm bis viele cm separiert wie bei anderen Technologien. Mit NV-Magnetometer-Einheiten, die nicht nur die Feldstärke, sondern auch die Richtung des Magnetfeldes bestimmen können, wird eine verbesserte Unterdrückung eines Hintergrundfeldes und somit die bessere Detektion von Signalen, die stark von Störsignalen überlagert werden, ermöglicht.

In einer Ausgestaltung weist die Vorrichtung eine Signalverarbeitungseinheit auf, mit der die wenigstens zwei NV-Magnetometereinheiten verbunden sind, wobei die Vorrichtung dazu eingerichtet ist, mittels der Signalverarbeitungseinheit eine effektive magnetische Feldstärke und/oder eine effektive magnetische Feldrichtung als Differenz der mittels der wenigstens zwei NV-Magnetometereinheiten erfassten magnetischen Feldstärke bzw. Feldrichtung zu bestimmen. Sowohl eine drahtlose als auch drahtgebundene Anbindung zwischen Sensorik und Signalverarbeitungseinheit ist vorgesehen.

Für die Anwendung wird eine Samplingrate benötigt, welche höher als das Herzsignal ist, um dieses aufzulösen, insbesondere größer als 50Hz. Dabei wird ein Bereich von 200 Hz bis 400 Hz als besonders vorteilhaft erachtet. Höher ist für die Auflösung immer besser, verschärft aber die Anforderungen an die Sensitivität.

Weitere Vorteile und Ausgestaltungen der Erfindung ergeben sich aus der Beschreibung und der beiliegenden Zeichnung.

Die Erfindung ist anhand von Ausführungsbeispielen in der Zeichnung schematisch dargestellt und wird im Folgenden unter Bezugnahme auf die Zeichnung beschrieben.

### Kurze Beschreibung der Zeichnungen

Figur 1 zeigt in einer schematischen Blockansicht die wesentlichen Komponenten eines NV-Zentren-Magnetometers, wie es im Rahmen der Erfindung Anwendung finden kann.
Figur 2 zeigt in verschiedenen Abbildungen a) bis c) jeweils in einer schematischen Blockansicht mögliche Anordnungen von NV-Magnetometereinheiten einer Vorrichtung zum Erfassen von magnetischen Signalen gemäß einer Ausgestaltung.
Figur 3 zeigt schematisch in einer Seitenansicht einen Benutzer auf einem Unterlagekörper gemäß einer Ausführungsform der Erfindung.
Figur 4 zeigt schematisch in drei Seitenansichten a) bis c) mögliche Ausgestaltungen von Unterlagekörpern gemäß Ausführungsformen der Erfindung.
Figur 5 zeigt schematisch in einer Seitenansicht mögliche Ausgestaltungen von Unterlagekörpern gemäß Ausführungsformen der Erfindung.
Figur 6 zeigt schematisch in vier Seitenansichten a) bis d) mögliche Ausgestaltungen von Unterlagekörpern gemäß Ausführungsformen der Erfindung.
Figur 7 zeigt schematisch in sechs Draufsichten a) bis f) mögliche Ausgestaltungen von Anordnungen mit einer oder mehreren NV-Magnetometereinheiten gemäß Ausführungsformen der Erfindung.
Figur 8 zeigt schematisch in vier Seitenansichten a) bis d) mögliche Ausgestaltungen von Vorrichtungen mit unterschiedlichen Unterlagekörpern mit einer oder mehreren Anordnungen mit NV-Magnetometereinheiten gemäß Ausführungsformen der Erfindung.
Figur 9 zeigt schematisch in zwei Seitenansichten a) und b) mögliche Ausgestaltungen von Vorrichtungen mit mehreren Anordnungen mit NV-Magnetometereinheiten und einer Signalverarbeitungseinheit gemäß Ausführungsformen der Erfindung.
Figur 10 zeigt schematisch in einer Seitenansicht eine Ausgestaltung einer Vorrichtung mit mehreren Anordnungen mit NV-Magnetometereinheiten, einer Signalverarbeitungseinheit und einer Hilfseinrichtung gemäß einer Ausführungsform der Erfindung.

### Ausführungsform(en) der Erfindung

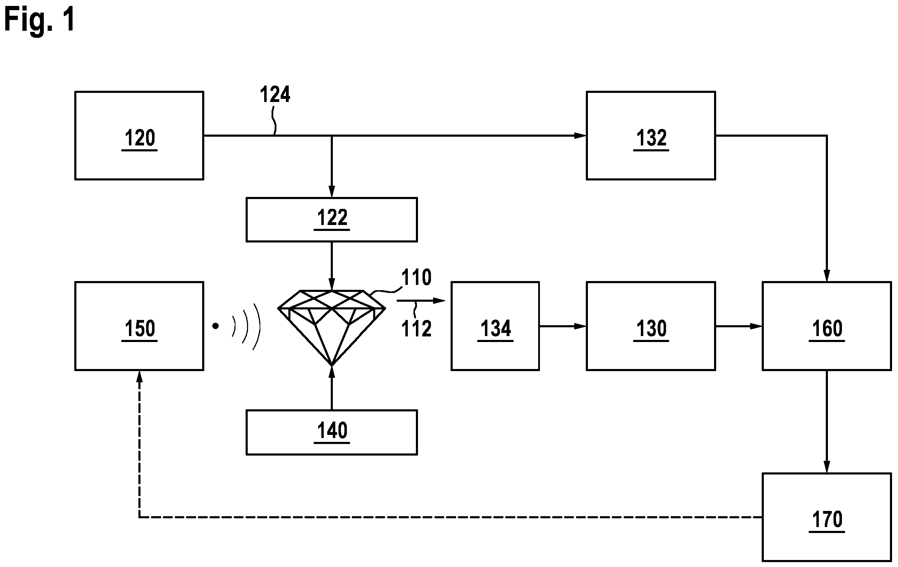

Figur 1 zeigt schematisch die wesentlichen Komponenten eines NV-Zentren-Magnetometers. Dabei ist zunächst ein Diamant 110 mit Stickstoff-Fehlstellen (NV) als Sensormedium vorhanden. Die optische Anregung der NV-Zentren kann durch eine geeignete Lichtquelle 120 wie etwa einen Pumplaser erreicht werden. Hier eignet sich beispielsweise ein frequenzverdoppelter Nd:YAG-Laser oder Halbleiterlaser im grünen Bereich von etwa 510-532nm, z.B. bei 532nm für eine off-resonance-Anregung. Alternativ können auch LEDs in geeigneten Wellenlängenbereichen genutzt werden. Je nach Anordnung kann das Licht der Lichtquelle 120 über geeignete optische Elemente 122 wie etwa Spiegel, Strahlteiler, fokussierende Optik wie Linsen und gegebenenfalls über faseroptische Elemente in den Diamanten 110 eingestrahlt werden. Außerdem kann das Anregungslicht durch den Laser kontinuierlich oder gepulst eingestrahlt werden, so dass beispielsweise Zeitfenster zur störungsfreien Fluoreszenzlichtmessung freigehalten werden.

Weiter kann das Magnetometer eine Mikrowellenquelle 150 umfassen, die in der Lage ist, ein elektromagnetisches Feld über eine Bandbreite hinweg, die die erwünschte Resonanzfrequenz abdeckt, im Sensormedium zu erzeugen, d.h. im Bereich der NV-Zentren des Diamanten 110. Eine Mikrowellen-Resonatorstruktur kann verwendet werden, um die erzeugten Mikrowellen über das Volumen des Messbereichs im Diamanten homogen zu verteilen. Die Resonatorstruktur bzw. die Mikrowellenquelle 150 ist dabei bevorzugt auf die Frequenz der Elektronenspinresonanzen gestimmt. Um Vektormagnetometrie zu ermöglichen, wird ein zusätzliches statisches Bias-Magnetfeld 140 erzeugt. Dadurch wird die Messung intrinsisch vektoriell. Dazu werden verschiedene Raumrichtungen in der Kristallstruktur verwendet. Zur Erzeugung eines solchen Magnetfelds 140 eignet sich beispielsweise eine Helmholtz-Spule, bei der mittels eines Spulenpaars ein im Wesentlichen homogenes Magnetfeld in einem begrenzten Bereich erzeugt werden kann.

Das entstehende Fluoreszenzlicht 112 aus dem Diamanten 110 kann wiederum über geeignete optische Elemente 134 wie etwa optische Filter, Strahlteiler, Linsen, und/oder faseroptische Elemente zu einem ersten Photodetektor 130 geleitet werden, der mindestens im Bereich der Fluoreszenzwellenlänge empfindlich ist. Der erste Photodetektor 130 kann auch unmittelbar an dem Diamanten 110 angeordnet sein. Ein zweiter Photodetektor 132 ist so angeordnet, dass er zumindest einen Teil des Anregungslichts der Lichtquelle 120 detektieren kann, welches beispielsweise durch einen Strahlteiler, einen Filter oder ein teildurchlässiges Element ausgekoppelt werden kann. Dieses Detektorsignal 132 des Anregungslichts kann als Referenzsignal verwendet werden, um beispielsweise durch Modulation des Anregungslichts mittels eines Lock-In-Verstärkers Hintergrundsignale zu eliminieren und das interessierende Resonanzsignal herauszustellen. Zusätzlich oder alternativ kann dieses Referenzsignal verwendet werden, um Schwankungen des Anregungslichts zu berücksichtigen. Entsprechende Schaltungen 160 wie ein Vorverstärker, ein logarithmischer Verstärker, ein Lock-In-Verstärker, Signalfilter oder andere sind also vorgesehen, um die Signale des ersten und des zweiten Photodetektors zu erhalten und die Signale auf geeignete Weise für die weitere Auswertung vorzuverarbeiten. Schließlich kann durch eine Signalverarbeitungseinheit 170 das vorverarbeitete Fluoreszenzsignal ausgewertet werden, z.B. mit einem geeigneten Mikrocontroller oder Prozessor, um aus dem Signal die gewünschten Parameter des detektierten Magnetfelds zu erhalten, insbesondere die Magnetfeldstärke und die Richtung des Magnetfelds.

Es versteht sich, dass eine solche Vorrichtung auch weitere, nicht gezeigte Einheiten aufweisen kann, wie Kommunikationseinheiten bzw. Schnittstellen zur Ausgabe der Messergebnisse. Eine solche Vorrichtung kann auch vorteilhaft in ein ASIC oder FPGA integriert sein.

Um in einer Alltagsumgebung einsetzbar zu sein, sollen Magnetfelder, die nicht von gewünschten schwachen Quellen stammen, aus der Messung möglichst eliminiert werden, insbesondere das Erdmagnetfeld im Bereich von 10⁻⁵ Tesla (einige Mikrotesla). Dagegen bewegen sich Herzmagnetfelder im Bereich von 10-100 mal 10⁻¹² Tesla (Picotesla).

Die Elimination der Hintergrundmagnetfelder kann durch eine Abschirmung oder durch eine Gradiometeranordnung bei der Magnetfeldmessung gemäß beispielhaften Ausführungsformen erreicht werden. Als Gradiometer werden grundsätzlich Sensoreinheiten bezeichnet, die in der Lage sind, nicht nur die Feldstärke, sondern auch den Gradienten des Felds zu erfassen.

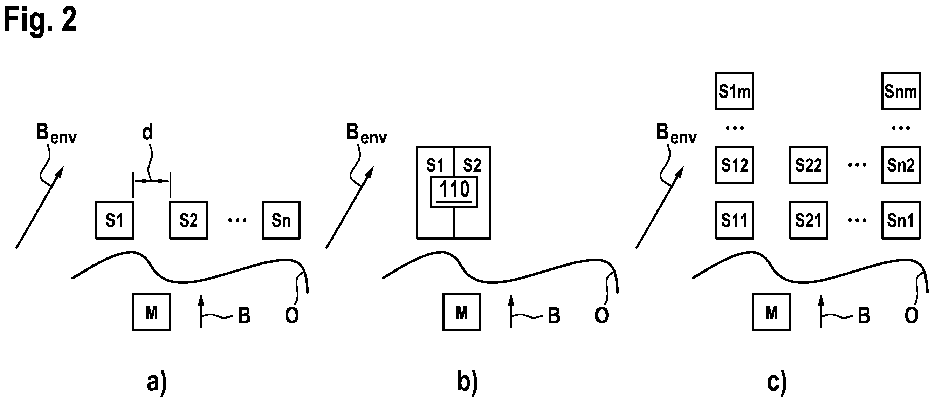

Dazu können mindestens zwei einzelne Magnetometereinheiten verwendet werden, die an räumlich unterschiedlichen Stellen angeordnet sind. Als Beispiel wird im Folgenden in Verbindung mit Figur 2 eine Sensoreinheit beschrieben, die zwei oder mehr NV-Zentren-Magnetometer in einer Gradiometeranordnung verwendet.

Figur 2 zeigt in verschiedenen Abbildungen a) bis c) mögliche geometrische Anordnungen von NV-Magnetometereinheiten einer Vorrichtung zum Erfassen von magnetischen Signalen gemäß einer Ausgestaltung. Abbildung a) zeigt in einer Seitenansicht eine Anordnung von wenigstens zwei NV-Magnetometereinheiten S1, S2, ..., Sn in einer beliebigen Anordnung zueinander in einer Ebene (senkrecht zur Zeichenebene, d.h. es ist nur die erste Reihe sichtbar). Abbildung b) zeigt in einer Seitenansicht zwei NV-Magnetometereinheiten S1, S2, deren Sensormedien Abschnitt desselben Diamantkristalls 110 sind. Abbildung c) zeigt in einer Seitenansicht eine Anzahl (n mal m) von NV-Magnetometereinheiten S11, S21, ..., Sn1, S12, S22, ..., Sn2, S1m, ... Snm in einer beliebigen dreidimensionalen Anordnung. Dabei schließen sich weitere Schichten hinter der Zeichenebene an, so dass insgesamt eine Art kubisches Gitter gebildet wird. Dabei ist wenigstens eine NV-Magnetometereinheit (nicht gezeigt), die z.B. in einer der hinteren Schichten liegt, nicht in der Ebene (Zeichenebene) angeordnet ist, in der andere NV-Magnetometereinheiten S11, S21, ..., Sn1, S12, S22, ..., Sn2, S1m, ... Snm angeordnet sind.

Weiterhin sind mit M eine Signalquelle, hier ein Herz, und mit O eine optionale Oberfläche (insbesondere Körperhaut), welche die Zugänglichkeit zur bzw. Erreichbarkeit der Magnetfeldquelle M begrenzt, bezeichnet.

In Ausgestaltungen der Erfindung können immer zwei NV-Magnetometereinheiten ein Gradiometer bilden, wobei dann - je nach Anzahl der NV-Magnetometereinheiten - insgesamt mehrere Gradiometer gebildet werden und das interessierende Signal erfassen. Daraus kann dann ein effektives Messsignal gebildet werden, insbesondere von der Signalverarbeitungseinheit, beispielsweise durch Mittelung, Summation usw.

Ein Abstand d zwischen zwei NV-Magnetometereinheiten S1, S2, ... oder genauer deren Sensormedien entspricht dem Abstand der Orte, an denen gleichzeitig Magnetfeldmessungen durchgeführt werden. Solange der Abstand der Messorte relativ klein ist, kann davon ausgegangen werden, dass die Stärke eines zusätzlichen Hintergrundmagnetfeldes Bₑₙᵥ an beiden Orten etwa gleich groß ist. Dagegen wird das interessierende schwache Magnetfeld B mit zunehmender Entfernung von der Magnetfeldquelle M deutlich abnehmen.

Indem also zwei NV-Magnetometereinheiten in unterschiedlichen Abständen von der Quelle bzw. vom Herz angeordnet werden, kann das Hintergrundfeld durch Bildung einer Differenz der erfassten Sensorwerte eliminiert werden und das interessierende kleine Magnetfeld bzw. dessen Gradient extrahiert werden: Da das magnetische Feld sich mit dem Quadrat des Abstands abschwächt, wird die größte Magnetfeldänderung durch die NV-Magnetometereinheiten in der Nähe der Quelle detektiert. Zu diesem Zweck können beispielsweise zwei NV-Magnetometereinheiten übereinander in einer axialen Gradiometerkonfiguration angeordnet werden, so dass jeweils ein NV-Magnetometereinheit einer ersten Schicht mit einer darunterliegenden NV-Magnetometereinheit einer zweiten, darunterliegenden Schicht, ein Gradiometer bildet. Durch eine weitere NV-Magnetometereinheit in einem großen Abstand, z.B. mindestens 1 m, zu den zwei NV-Magnetometereinheiten kann ebenfalls das Hintergrundfeld bestimmt werden.

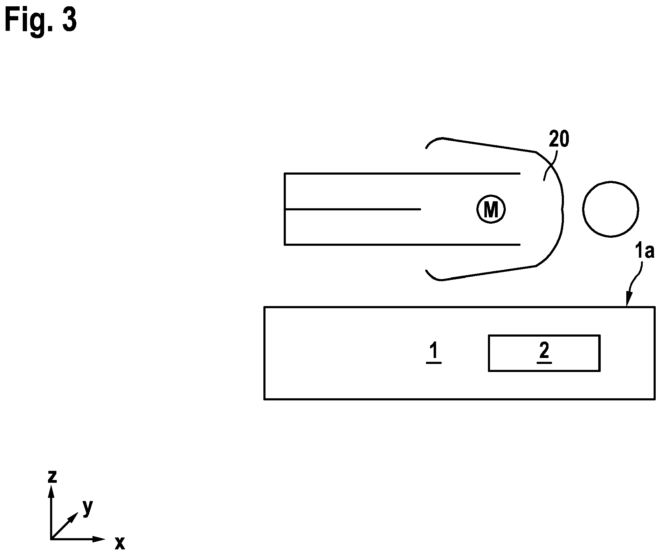

In den Figuren 3 bis 10 sind mögliche Ausführungsformen der Erfindung schematisch dargestellt und werden im Folgenden übergreifend beschrieben. Gleiche Elemente sind dabei mit gleichen Bezugszeichen versehen und werden nicht mehrfach beschrieben.

Dabei ist jeweils eine Vorrichtung 2 zum Erfassen von magnetischen Signalen gezeigt, die einen Unterlagekörper 1 mit einer Auflagefläche 1a und wenigstens eine Anordnung 3 aus wenigstens zwei Stickstoff-Fehlstellen-Zentren-, NV-, Magnetometereinheiten 4 aufweist, wobei die wenigstens eine Anordnung 3 in dem Unterlagekörper 1 eingebettet ist. Der Unterlagekörper ist dazu eingerichtet, einen Benutzer 20 sitzend oder liegend auf der Auflagefläche aufzunehmen. Die Vorrichtung 2 dient zum Erfassen von magnetischen Signalen, die von einem schlagenden Herz (M) erzeugt werden, kann jedoch grundsätzlich alle magnetischen Signale erfassen, insbesondere Biosignale, also solche, die von Lebewesen ausgehen. Zur Veranschaulichung weisen die Figuren jeweils oben links ein Koordinatensystem auf, wobei die Zeichenebene die x-z-Ebene darstellt und die y-Achse in die Zeichenebene hinein verläuft.

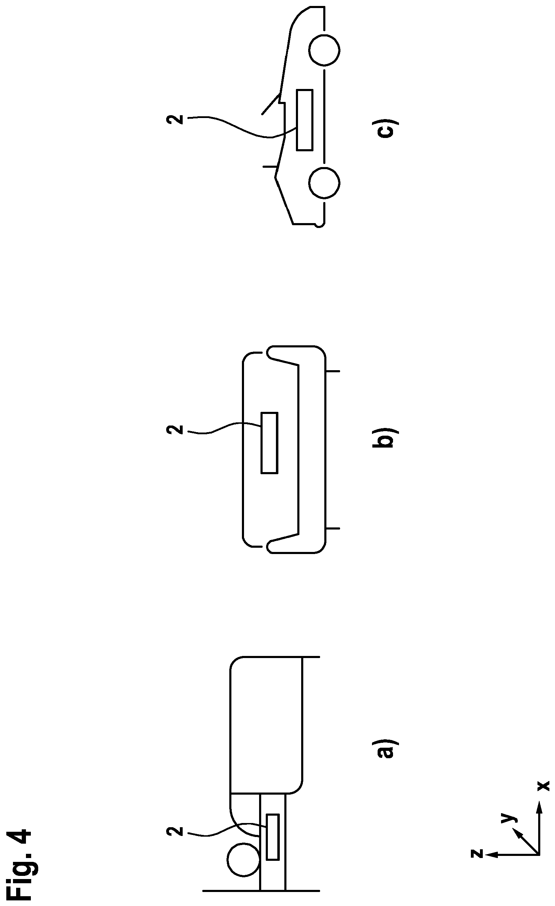

In Figur 3 ist eine Matratze als Unterlagekörper 1 gezeigt, in Figur 4a) eine Matratze in einem Bett, in Figur 4b) ein Sofa und in Figur 4c) ein Autositz.

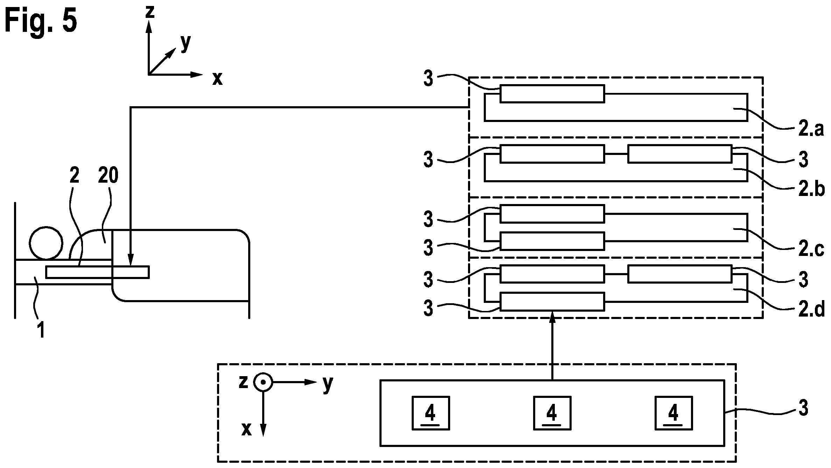

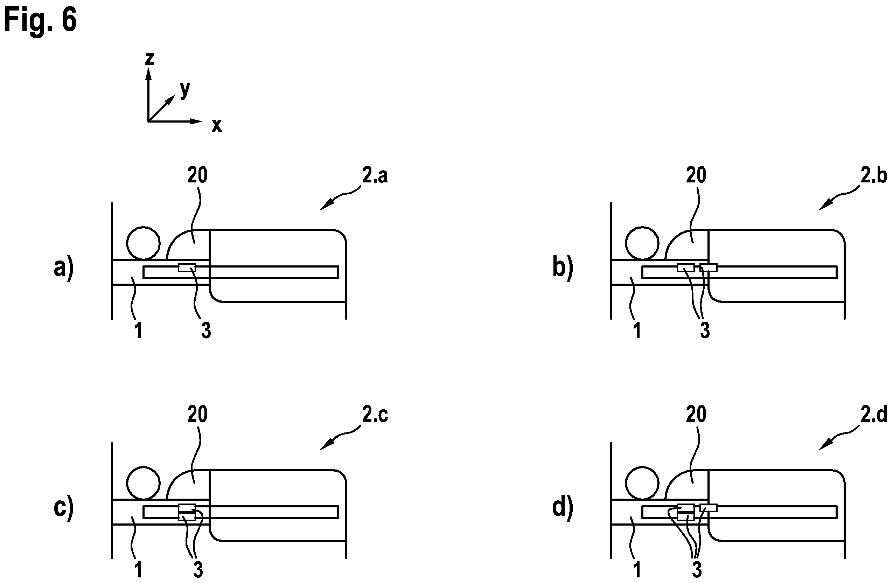

Figur 5 zeigt in einer schematischen Seitenansicht eine ausgedehnte Vorrichtung 2 in einer Matratze eines Bettes mit einem Benutzer, wie sie für eine Langzeitüberwachung insbesondere von magnetischen Herzsignalen eingesetzt werden kann. Rechts in Figur 5 und in Figur 6 sind verschiedene Varianten 2.a bis 2.d gezeigt, wie eine oder mehrere Anordnungen 3 aus NV-Magnetometereinheiten 4 in einer Vorrichtung 2 angeordnet sein können. Eine Vorrichtung kann dabei eine Anordnung (Variante 2.a) oder mehr als eine Anordnung (Varianten 2.b bis 2.d) aufweisen. Die Anordnungen können ebenfalls in einer bestimmten geometrischen Anordnung angeordnet sein, beispielsweise ein einer Linie (1D), Ebene (2D) oder im Raum verteilt (3D). Rechts unten in Figur 5 ist ein Schema einer Anordnung 3 mit mehreren NV-Magnetometereinheiten 4 in Draufsicht gezeigt, die selbst ebenfalls in einer geometrischen Anordnung, hier als Linie, angeordnet sind. Die NV-Magnetometereinheiten 4 einer Anordnung 3 können selbst ebenfalls in einer bestimmten geometrischen Anordnung angeordnet sein, beispielsweise in einer Linie (1D), Ebene (2D) oder im Raum verteilt (3D), wie auch bereits im Zusammenhang mit Figur 2 erläutert. Wie erläutert, können dabei immer zwei NV-Magnetometereinheiten ein Gradiometer bilden, wobei dann - je nach Anzahl der NV-Magnetometereinheiten - insgesamt mehrere Gradiometer gebildet werden und das interessierende Signal erfassen. Daraus kann dann ein effektives Messsignal gebildet werden, insbesondere von der Signalverarbeitungseinheit, beispielsweise durch Mittelung, Summation usw.

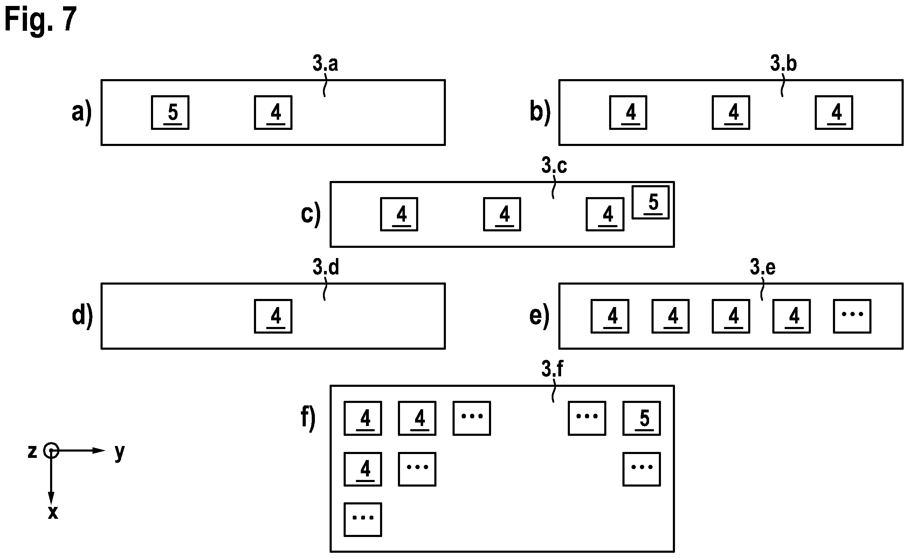

Figur 7 zeigt in schematischer Draufsicht in verschiedenen Ansichten a) bis f) Varianten 3.a bis 3.f von Anordnungen 3 mit jeweils einer oder mehreren NV-Magnetometereinheiten 4, jeweils mit keinem, einem oder mehreren weiteren Sensoren 5. Bei den Sensoren 5 kann es sich insbesondere um Drucksensoren, Pulsoxymeter, Temperatursensoren usw. handeln. Die NV-Magnetometereinheiten 4 und/oder die Sensoren 5 einer Anordnung 3 können in einer bestimmten geometrischen Anordnung angeordnet sein, beispielsweise ein einer Linie (1D), Ebene (2D) oder im Raum verteilt (3D), wie auch bereits im Zusammenhang mit Figur 2 oder 5 erläutert.

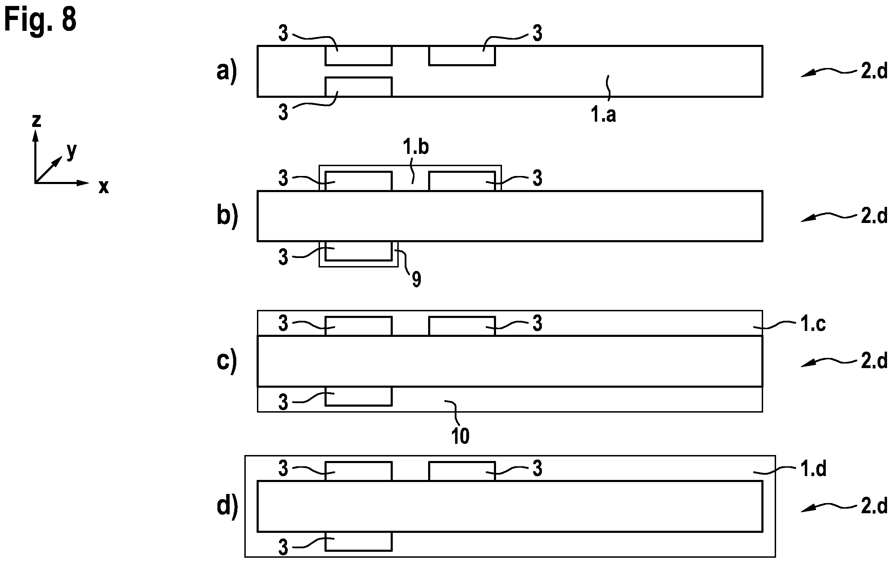

In Figur 8 sind in vier Seitenansichten a) bis d) verschiedene Varianten 2.d einer Vorrichtung 2 mit zwei Anordnungen 3 im Bereich einer Oberseite und einer Anordnung 3 im Bereich einer Unterseite eines Unterlagekörpers gezeigt. In Variante a) sind die drei Anordnungen 3 in einer Matratze 1.a als Unterlagekörper eingebettet. In Variante b) sind zwei Anordnungen 3 in einem Kissen 1.b als Unterlagekörper eingebettet. Zusätzlich ist eine Anordnung unter der Matratze, z.B. in einer Unterlage 9 angeordnet. In Variante c) sind zwei Anordnungen 3 in einem Topper 1.c als Unterlagekörper eingebettet. Zusätzlich ist eine Anordnung unter der Matratze, z.B. in einem Lattenrost 10 angeordnet. In Variante d) sind die drei Anordnungen 3 in einem Matratzenüberzeug 1.d als Unterlagekörper eingebettet. Zweckmäßigerweise kann hier auf verschiedene, der Bequemlichkeit dienende, Mechanismen zurückgegriffen werden, z.B. Schichten, z.B. Schaumstoff, z.B. Bezüge, z.B. verschiedene Umhüllungsmaterialien, z.B. Materialien zum Schutz der Elektronik aber auch zur Abschirmung und Komforterhöhung.

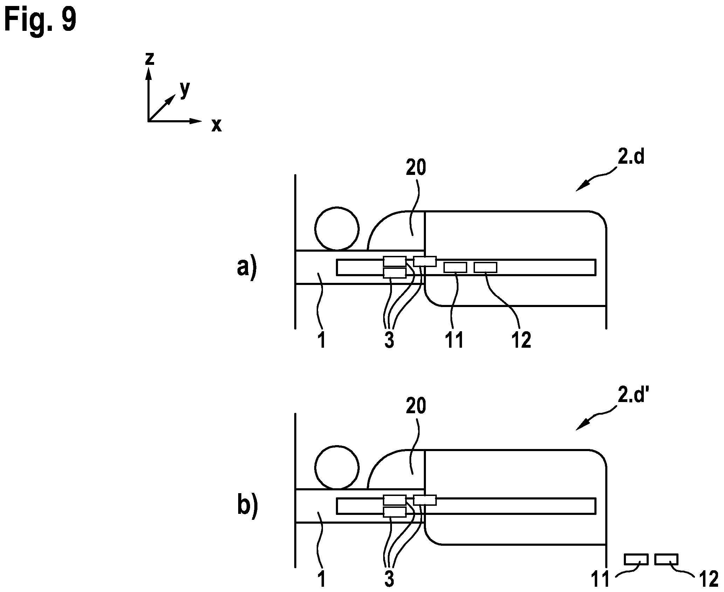

In Figur 9 sind in zwei Seitenansichten a) und b) verschiedene Varianten 2.d, 2.d' einer Vorrichtung 2 mit zwei Anordnungen 3 im Bereich einer Oberseite und einer Anordnung 3 im Bereich einer Unterseite eines Unterlagekörpers 1, insbesondere einer Matratze, gezeigt. Weiterhin weist die Vorrichtung eine Signalverarbeitungseinheit 11 auf, mit der die NV-Magnetometereinheiten der Anordnungen 3 verbunden sind, um eine effektive magnetische Feldstärke und/oder Feldrichtung zu bestimmen. Weiterhin kann eine Kommunikationseinheit 12 vorgesehen sein, um die Vorrichtung 2 mit anderen Geräten wie einem PC, Tablet-PC, Smartphone zur Ein- und Ausgabe und Bedienung zu verbinden. Die Kommunikationseinheit 12 kann z.B. kabelgebundene und/oder drahtlose Schnittstellen aufweisen. Dabei sind in Variante 2.d die Signalverarbeitungseinheit 11 und Kommunikationseinheit 12 ebenfalls in dem Unterlagekörper integriert, und in Variante 2.d' außerhalb vom Unterlagekörper angeordnet.

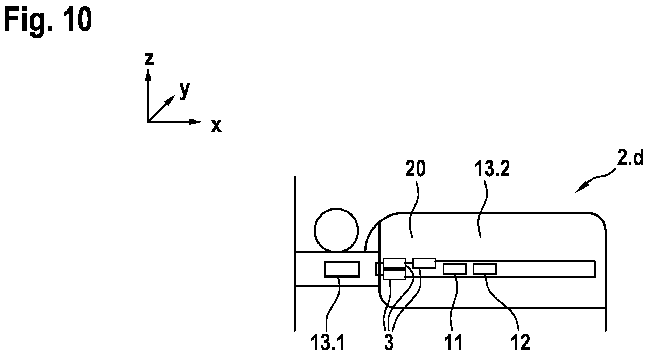

In Figur 10 ist schematisch in einer Seitenansicht eine Ausgestaltung einer Vorrichtung 2.d mit mehreren Anordnungen 3 mit NV-Magnetometereinheiten, einer Signalverarbeitungseinheit 11, einer Kommunikationseinheit 12 und zweier Varianten von Hilfseinrichtungen 13.1, 13.2 gemäß Ausführungsformen der Erfindung gezeigt.

Die Hilfseinrichtung 13.1, 13.2 kann wenigstens eine Funktion erfüllen, ausgewählt aus einer Funktion zum Ableiten von Abwärme, zur Wärmeschirmung, zur Wärmeleitung, zur Magnetfeldkompensation (z.B. aktiv durch Spulen), zur (elektro-)magnetischen Abschirmung, zum Schutz vor Nässe und zur Komforterhöhung (Verwendung bestimmter Verpackungs- und Verbundstoffe, um den Schlaf angenehm und komfortabel zu gestalten). Die Hilfseinrichtung 13.1, 13.2 kann eine Struktur, beispielsweise ein Netz, aus einem ferromagnetischen Material mit hoher magnetischer Permeabilität z.B. größer 100, aufweisen.

Die Hilfseinrichtung 13.1 kann ebenfalls in den Unterlagekörper eingebettet sein. Sie kann auch unter dem Unterlagekörper, z.B. in einem Bettgestell oder Lattenrost, oder in einen Auflagekörper 13.2 auf dem Benutzer 20, z.B. in Form einer Zudecke, eingebettet sein.

## Patentansprüche

1. Vorrichtung (2) zum Erfassen von magnetischen Signalen, die von einem schlagenden Herz (M) erzeugt werden, aufweisend
einen Unterlagekörper (1) mit einer Auflagefläche (1a), und
eine Anordnung (3) aus wenigstens zwei Stickstoff-Fehlstellen-Zentren-, NV, Magnetometereinheiten (S1, S2, ..., Sn; S11, S21, ..., Sn1, S12, S22, ..., Sn2, S1m, ... Snm; 4), wobei die Anordnung in dem Unterlagekörper (1) eingebettet ist,
wobei der Unterlagekörper (1) dazu eingerichtet ist, einen Benutzer (20) sitzend oder liegend auf der Auflagefläche (1a) aufzunehmen und wobei die wenigstens zwei NV-Magnetometereinheiten (S1, S2, ..., Sn; S11, S21, ..., Sn1, S12, S22, ..., Sn2, S1m, ... Snm; 4) wenigstens vier NV-Magnetometereinheiten (4) umfassen und wobei die Anordnung (3) eine dreidimensionale Anordnung (3) ist, bei der wenigstens eine der wenigstens vier NV-Magnetometereinheiten (4) nicht in einer Ebene angeordnet ist, in der wenigstens drei andere der wenigstens vier NV-Magnetometereinheiten (4) angeordnet sind.

2. Vorrichtung nach Anspruch 1, wobei der Unterlagekörper (1) elastisches Material zwischen der Anordnung (3) und der Auflagefläche (1a) aufweist.

3. Vorrichtung nach Anspruch 1 oder 2, wobei der Unterlagekörper (1) ein Polster, eine Matratze, eine Liege, eine Matte, ein Bett, ein Sitz oder ein Stuhl ist.

4. Vorrichtung nach einem der vorstehenden Ansprüche, aufweisend eine Struktur aus einem Material mit magnetischer Permeabilität größer 1 auf einer der Auflagefläche (1a) abgewandten Seite der Anordnung (3), und/oder einen Auflagekörper (13.2) enthaltend die Struktur.

5. Vorrichtung nach einem der vorstehenden Ansprüche, die dazu eingerichtet ist, mittels jeder der wenigstens zwei NV-Magnetometereinheiten (S1, S2, ..., Sn; S11, S21, ..., Sn1, S12, S22, ..., Sn2, S1m, ... Snm; 4) eine magnetische Feldstärke und Feldrichtung zu erfassen.

6. Vorrichtung nach einem der vorstehenden Ansprüche, mit einer Signalverarbeitungseinheit (170, 11), mit der die wenigstens zwei NV-Magnetometereinheiten (S1, S2, ..., Sn; S11, S21, ..., Sn1, S12, S22, ..., Sn2, S1m, ... Snm; 4) verbunden sind,
wobei die Vorrichtung dazu eingerichtet ist, mittels der Signalverarbeitungseinheit (170, 11) eine effektive magnetische Feldstärke und/oder Feldrichtung als Differenz von mittels der wenigstens zwei NV-Magnetometereinheiten (S1, S2, ..., Sn; S11, S21, ..., Sn1, S12, S22, ..., Sn2, S1m, ... Snm; 4) erfassten magnetischen Feldstärken bzw. Feldrichtungen zu bestimmen.

7. Vorrichtung nach einem der vorstehenden Ansprüche, wobei die Anordnung (3) eine zweidimensionale Anordnung (3) ist, bei der die wenigstens zwei NV-Magnetometereinheiten (S1, S2, ..., Sn; S11, S21, ..., Sn1, S12, S22, ..., Sn2, S1m, ... Snm; 4) in einer Ebene angeordnet sind.

8. Vorrichtung nach einem der vorstehenden Ansprüche, wobei jede der wenigstens zwei NV-Magnetometereinheiten (S1, S2, ..., Sn; S11, S21, ..., Sn1, S12, S22, ..., Sn2, S1m, ... Snm; 4) als Sensormedium (110) einen Diamantkristall oder einen Abschnitt eines Diamantkristalls mit Stickstoff-Fehlstellen-Zentren aufweist, wobei die Vorrichtung dazu eingerichtet ist, eine magnetische Feldstärke und/oder Feldrichtung durch Auslesen einer von der magnetischen Feldstärke abhängigen Spinresonanz in dem Sensormedium (110) zu erfassen.

9. Vorrichtung nach Anspruch 8, weiterhin aufweisend mindestens eine Anregungslichtquelle (120) zum Einstrahlen von Licht (124) in das Sensormedium (110), mindestens eine Mikrowellenquelle (150) zum Erzeugen eines resonanten Felds in dem Sensormedium sowie mindestens einen Photodetektor (130) zum Erfassen von resonanzabhängigem Fluoreszenzlicht (112) aus dem Sensormedium (110).

10. Vorrichtung nach Anspruch 9, wobei der wenigstens zwei NV-Magnetometereinheiten (S1, S2, ..., Sn; S11, S21, ..., Sn1, S12, S22, ..., Sn2, S1m, ... Snm; 4) dieselbe Anregungslichtquelle (120) und/oder dieselbe Mikrowellenquelle (150) zugeordnet sind.

11. Vorrichtung nach einem der Ansprüche 8 bis 10, wobei das Sensormedium der wenigstens zwei NV-Magnetometereinheiten (S1, S2, ..., Sn; S11, S21, ..., Sn1, S12, S22, ..., Sn2, S1m, ... Snm; 4) jeweils einen Abschnitt desselben Diamantkristalls (110) aufweist.

12. Vorrichtung nach einem der Ansprüche 8 bis 11, wobei der Abstand (d) zwischen den Sensormedien (110) der wenigstens zwei NV-Magnetometereinheiten (S1, S2, ..., Sn; S11, S21, ..., Sn1, S12, S22, ..., Sn2, S1m, ... Snm; 4) zwischen 1 und 30 Millimetern beträgt, bevorzugt zwischen 5 und 20 Millimetern.

13. Vorrichtung nach einem der vorstehenden Ansprüche, weiterhin aufweisend eine weitere NV-Magnetometereinheit (S1, S2, ..., Sn; S11, S21, ..., Sn1, S12, S22, ..., Sn2, S1m, ... Snm; 4), die von den wenigstens zwei NV-Magnetometereinheiten (S1, S2, ..., Sn; S11, S21, ..., Sn1, S12, S22, ..., Sn2, S1m, ... Snm; 4) mindestens 1 m beabstandet ist.

## Claims

1. Device (2) for detecting magnetic signals generated by a beating heart (M), comprising
a support body (1) having a contact surface (1a), and
an arrangement (3) consisting of at least two nitrogen vacancy centre, NV, magnetometer units (S1, S2, ..., Sn; S11, S21, ..., Sn1, S12, S22, ..., Sn2, S1m, ... SNm; 4), the arrangement being embedded in the support body (1),
the support body (1) being designed to accommodate a user (20) sitting or lying on the contact surface (1a), and the at least two NV magnetometer units (S1, S2, ..., Sn; S11, S21, ..., Sn1, S12, S22, ..., Sn2, S1m, ... Snm; 4) comprising at least four NV magnetometer units (4), and the arrangement (3) being a three-dimensional arrangement (3) in which at least one of the at least four NV magnetometer units (4) is not arranged in a plane in which at least three other of the at least four NV magnetometer units (4) are arranged.

2. Device according to Claim 1, the support body (1) having elastic material between the arrangement (3) and the support surface (1a).

3. Device according to Claim 1 or 2, the support body (1) being a cushion, a mattress, a couch, a mat, a bed, a seat or a chair.

4. Device according to one of the preceding claims, comprising a structure made of a material having magnetic permeability greater than 1 on a side of the arrangement (3) that faces away from the contact surface (1a), and/or a support body (13.2) containing the structure.

5. Device according to one of the preceding claims, designed to use each of the at least two NV magnetometer units (S1, S2, ..., Sn; S11, S21, ..., Sn1, S12, S22, ..., Sn2, S1m, ... Snm; 4) to detect a magnetic field strength and field direction.

6. Device according to one of the preceding claims, having a signal processing unit (170, 11) to which the at least two NV magnetometer units (S1, S2, ..., Sn; S11, S21, ..., Sn1, S12, S22, Sn2, S1m, ... Snm; 4) are connected, the device being designed to use the signal processing unit (170, 11) to determine an effective magnetic field strength and/or field direction as the difference between magnetic field strengths or field directions detected by means of the at least two NV magnetometer units (S1, S2, ..., Sn; S11, S21, ..., Sn1, S12, S22, ..., Sn2, S1m, ... Snm; 4).

7. Device according to one of the preceding claims, the arrangement (3) being a two-dimensional arrangement (3) in which the at least two NV magnetometer units (S1, S2, ..., Sn; S11, S21, ..., Sn1, S12, S22, ..., Sn2, S1m, ... Snm; 4) are arranged in one plane.

8. Device according to one of the preceding claims, each of the at least two NV magnetometer units (S1, S2, ..., Sn; S11, S21, ..., Sn1, S12, S22, ..., Sn2, S1m, ... Snm; 4) comprising as the sensor medium (110) a diamond crystal or a portion of a diamond crystal having nitrogen vacancy centres, the device being designed to detect a magnetic field strength and/or field direction by reading a spin resonance in the sensor medium (110) that is dependent on the magnetic field strength.

9. Device according to Claim 8, additionally comprising at least one excitation light source (120) for radiating light (124) into the sensor medium (110), at least one microwave source (150) for generating a resonant field in the sensor medium, and at least one photodetector (130) for detecting resonance-dependent fluorescent light (112) from the sensor medium (110).

10. Device according to Claim 9, the at least two NV magnetometer units (S1, S2, ..., Sn; S11, S21, ..., Sn1, S12, S22, ..., Sn2, S1m, ... Snm; 4) having the same associated excitation light source (120) and/or the same associated microwave source (150).

11. Device according to one of Claims 8 to 10, the sensor medium of each of the at least two NV magnetometer units (S1, S2, ..., Sn; S11, S21, ..., Sn1, S12, S22, ..., Sn2, S1m, ... Snm; 4) comprising a portion of the same diamond crystal (110).

12. Device according to one of Claims 8 to 11, the distance (d) between the sensor media (110) of the at least two NV magnetometer units (S1, S2, ..., Sn; S11, S21, ..., Sn1, S12, S22, ..., Sn2, S1m, ... Snm; 4) being between 1 and 30 millimetres, preferably between 5 and 20 millimetres.

13. Device according to one of the preceding claims, additionally comprising a further NV magnetometer unit (S1, S2, ..., Sn; S11, S21, ..., Sn1, S12, S22, ..., Sn2, S1m, ... Snm; 4), which is at a distance of at least 1 m from the at least two NV magnetometer units (S1, S2, ..., Sn; S11, S21, ..., Sn1, S12, S22, ..., Sn2, S1m, ... Snm; 4).

## Revendications

1. Dispositif (2) pour la détection de signaux magnétiques générés par un cœur battant (M), comprenant
un corps de sous-couche (1) avec une surface d'appui (1A), et
un agencement (3) d'au moins deux unités de magnétomètre à centres à lacunes d'azote, NV, (S1, S2, ..., Sn ; S11, S21, ..., Sn1, S12, S22, ..., Sn2, S1m, ... Snm ; 4), l'agencement étant encastré dans le corps de sous-couche (1),
le corps de sous-couche (1) étant configuré pour recevoir un utilisateur (20) en position assise ou allongée sur la surface d'appui (1a), et les au moins deux unités de magnétomètre NV (S1, S2, ..., Sn ; S11, S21, ..., Sn1, S12, S22, ..., Sn2, S1m, ... Snm ; (4) comprenant au moins quatre unités de magnétomètre NV (4), et l'agencement (3) étant un agencement tridimensionnel (3), dans lequel au moins une des au moins quatre unités de magnétomètre NV (4) n'est pas disposée dans un plan dans lequel au moins trois autres des au moins quatre unités de magnétomètre NV (4) sont disposées.

2. Dispositif selon la revendication 1, dans lequel le corps de sous-couche (1) présente un matériau élastique entre l'agencement (3) et la surface d'appui (1a).

3. Dispositif selon la revendication 1 ou 2, dans lequel le corps de sous-couche (1) est un coussin, un matelas, une couchette, un tapis, un lit, un siège ou une chaise.

4. Dispositif selon l'une des revendications précédentes, comprenant une structure réalisée en un matériau ayant une perméabilité magnétique supérieure à 1, disposée sur un côté de l'agencement (3) opposé à la surface d'appui (1a), et/ou comprenant un corps d'appui (13.2) contenant la structure.

5. Dispositif selon l'une des revendications précédentes, configuré pour, au moyen de chacune des au moins deux unités de magnétomètre NV (S1, S2, ..., Sn ; S11, S21, ..., Sn1, S12, S22, ..., Sn2, S1m, ... Snm ; 4) détecter une intensité de champ magnétique et une direction de champ.

6. Dispositif selon l'une des revendications précédentes, comprenant une unité de traitement du signal (170, 11), à laquelle les au moins deux unités de magnétomètre NV (S1, S2, ..., Sn ; S11, S21, ..., Sn1, S12, S22, Sn2, S1m, ... Snm ; 4) sont reliées,
le dispositif étant configuré pour, au moyen de l'unité de traitement du signal (170, 11), déterminer une intensité de champ magnétique effective et/ou une direction de champ en tant que différence entre des intensités de champ magnétique et/ou des directions de champ détectées au moyen des au moins deux unités de magnétomètre NV (S1, S2, ..., Sn ; S11, S21, ..., Sn1, S12, S22, ..., Sn2, S1m, ... Snm ; 4).

7. Dispositif selon l'une des revendications précédentes, dans lequel l'agencement (3) est un agencement bidimensionnel (3), dans lequel les au moins deux unités de magnétomètre NV (S1, S2, ..., Sn ; S11, S21, ..., Sn1, S12, S22, ..., Sn2, S1m, ... Snm ; 4) sont disposées dans un plan.

8. Dispositif selon l'une des revendications précédentes, dans lequel chacune des au moins deux unités de magnétomètre NV (S1, S2, ..., Sn; S11, S21, ..., Sn1, S12, S22, ..., Sn2, S1m, ... Snm ; 4) comporte, en tant que milieu capteur (110), un cristal de diamant ou une portion de cristal de diamant présentant des centres à lacunes d'azote, le dispositif étant configuré pour détecter une intensité de champ magnétique et/ou une direction de champ par lecture d'une résonance de spin dépendante de l'intensité du champ magnétique dans le milieu capteur (110).

9. Dispositif selon la revendication 8, comprenant en outre au moins une source de lumière d'excitation (120) destinée à irradier de la lumière (124) dans le milieu capteur (110), au moins une source de micro-ondes (150) destinée à générer un champ résonant dans le milieu capteur, ainsi qu'au moins un photodétecteur (130) destiné à détecter une fluorescence dépendante de la résonance (112) provenant du milieu capteur (110).

10. Dispositif selon la revendication 9, dans lequel les au moins deux unités de magnétomètre NV (S1, S2, ..., Sn ; S11, S21, ..., Sn1, S12, S22, ..., Sn2, S1m, ... Snm ; 4) sont associées la même source de lumière d'excitation (120) et/ou la même source de micro-ondes (150).

11. Dispositif selon l'une des revendications 8 à 10, dans lequel le milieu capteur des au moins deux unités de magnétomètre NV (S1, S2, ..., Sn ; S11, S21, ..., Sn1, S12, S22, ..., Sn2, S1m, ... Snm ; 4) présente respectivement une portion d'un même cristal de diamant (110).

12. Dispositif selon l'une des revendications 8 à 11, dans lequel la distance (d) entre les milieux capteurs (110) des au moins deux unités de magnétomètre NV (S1, S2, ..., Sn ; S11, S21, ..., Sn1, S12, S22, ..., Sn2, S1m, ... Snm ; 4) est comprise entre 1 et 30 millimètres, de préférence entre 5 et 20 millimètres.

13. Dispositif selon l'une des revendications précédentes, comprenant en outre une autre unité de magnétomètre NV (S1, S2, ..., Sn ; S11, S21, ..., Sn1, S12, S22, ..., Sn2, S1m, ... Snm ; 4), qui est espacée d'au moins 1 m des au moins deux unités de magnétomètre NV (S1, S2, ..., Sn ; S11, S21, ..., Sn1, S12, S22, ..., Sn2, S1m, ... Snm ; 4).
